# EUROPEAN PATENT SPECIFICATION

(11) **EP 4 136 068 B1**
(45) Date of publication and mention of the grant of the patent: **10.07.2024**
(21) Application number: 21726051.2
(22) Date of filing: 12.04.2021
(51) Int. Cl.: C07C 255/50

(54) **A PROCESS FOR THE PREPARATION OF 4-CYANOBENZOYL CHLORIDES**
VERFAHREN ZUR HERSTELLUNG VON 4-CYANOBENZOYL-CHLORIDEN
PROCÉDÉ DE PRÉPARATION DE CHLORURES 4-CYANOBENZOYL

(30) Priority: 16.04.2020 IN 202021016414; 16.06.2020 EP 20180312
(43) Date of publication of application: 22.02.2023
(73) Proprietor: BASF SE, 67056 Ludwigshafen am Rhein (DE)
(72) Inventor: VOGT, Florian, 67056 Ludwigshafen (DE); GEBHARDT, Joachim, 67056 Ludwigshafen (DE); BORATE, Kailaskumar, Mumbai 400705 (IN); WOLF, Bernd, 67056 Ludwigshafen (DE); SUTORIS, Heinz Friedrich, 67117 Limburgerhof (DE); GOETZ, Roland, 67056 Ludwigshafen (DE)
(74) Representative: BASF IP Association
(86) International application number: PCT/EP2021/059424
(87) International publication number: WO 2021/209377

(56) References cited:
- WO-A2-2011/018170
- CN-A- 109 942 416
- US-A1- 2008 119 457
- US-A1- 2010 317 643
- C. K. BRADSHER ET AL: "Reactions in the m-Terphenyl Series 1", JOURNAL OF THE AMERICAN CHEMICAL SOCIETY, vol. 72, no. 9, 1 September 1950 (1950-09-01), US, pages 4189 - 4192, XP055751898, ISSN: 0002-7863, DOI: 10.1021/ja01165a100

## Description

The present invention relates to a process for the preparation of 4-cyanobenzoyl chlorides of formula I through reaction of compounds of formula II with a chlorinating agent.

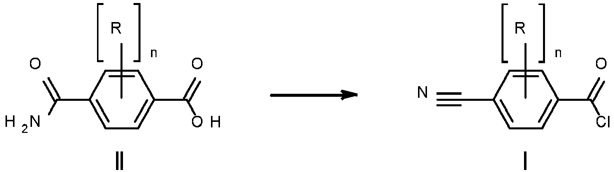

4-Cyanobenzoyl chlorides of formula I are versatile and highly reactive synthetic intermediates. The functional groups offer various strategic options for orthogonal synthesis concepts taking advantage of the differentiated reactivity of the nitrile and the carboxylic acid chloride group towards a large number of reactants/reagents. For example, compounds of formula I can be employed in the efficient preparation of known benzamide type trifluoromethyl-1,2,4-oxadiazoles, for example compounds disclosed in WO 2015/185485 A1 and WO 2017/211649 A1, which are useful for controlling phytopathogenic fungi. CN 109942416 discloses a process for preparing benzoyl chlorides by reaction of an aryl carboxylic acid with phosphorus trichloride.

Synthetic access to 4-cyanobenzoyl chlorides of formula I via 4-carbamoylbenzoic acids of type II is particularly attractive since it taps into industrial feedstock such as terephthalic acid dichloride, which is available on industrial scale at low price. For instance, desymmetrization of terephthalic acid dichloride affords compounds of formula II, wherein R is hydrogen, in two simple steps.

It is part of the common general knowledge of the skilled person in the art of synthetic chemistry that carboxylic acids react with suitable chlorinating agents to yield carboxylic acid chlorides. Likewise, it is widely known that primary carboxamides can undergo dehydration with the same type of chlorinating agents to obtain the corresponding nitriles. However, the prior art does not report selective transformations of compounds featuring both these functional groups in one molecule, and to transform each functional group in one step using one type of chlorinating agent.

The skilled person in the art knows that carboxylic acid halides are highly reactive electrophiles, which react non-selectively with nucleophilic species, for example with primary carboxamides. In the same sense the reaction of a primary carboxamide that eventually leads to the formation of a nitrile proceeds *via* highly reactive intermediate species that are prone to intermolecular reactions. Given these properties, the skilled person would expect a great number of side reactions when using chlorinating agents with bifunctional compounds of formula II, including, amongst others, the undesired formation of oligomers and/or polymers. Accordingly, the skilled person would not have expected any appreciable conversion of compounds II in favor of the desired compounds of formula I and he would not have considered to use them in large-scale processes for the preparation of 4-cyanobenzoyl chlorides of formula I.

The inventors surprisingly found that against all odds moderate to good yields of compounds I can be obtained by reaction of compounds II with chlorinating agents. This process provides an economic process enabling the efficient preparation of compounds of formula I on an industrial scale in high yield and with low amounts of side-products.

Accordingly, the present invention relates to a process for preparing 4-cyanobenzoyl chlorides of formula I, 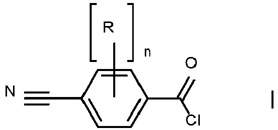 wherein
- R: is halogen, C₁-C₄-alkyl, C₁-C₄-haloalkyl, C₁-C₄-alkoxy, or C₁-C₄-haloalkoxy;
- n: is 0, 1 or 2;
the process comprising reacting a compound of formula II, 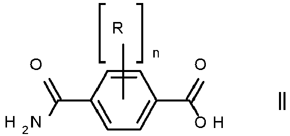 wherein the variable R is as defined above for compounds of formula I, with a chlorinating agent.

In one embodiment the chlorinating agent of the process of the invention is selected from the group consisting of phosphoryl trichloride, trichlorophosphane, pentachlorophosphane, thionyl chloride, phosgene, diphosgene, triphosgene, and oxalyl chloride; in a preferred embodiment the chlorinating agent is phosphoryl trichloride.

Typically, the amount of the chlorinating agent is between 2 and 15 equivalents, preferably between 2 and 10 equivalents, more preferably between 2 and 8 equivalents, based on the amount of compound II.

The process proceeds even faster in the presence of small amounts of *N*,*N*-dimethylformamide or N,N-dimethylacetamide. Therefore, in one aspect of the present invention the process is conducted in the presence of substoichiometric amounts of *N*,*N*-dimethylformamide or *N,N-*dimethylacetamide, preferably *N,N*-dimethylformamide, based on the amount of compound II. In one aspect *N,N*-dimethylformamide or *N,N*-dimethylacetamide, preferably *N,N-*dimethylformamide, is used in an amount of up to 0.5 equivalents, up to 0.2 equivalents, or up to 0.1 equivalents, based on the amount of compound II. In another aspect *N,N-*dimethylformamide or *N,N*-dimethylacetamide, preferably *N,N*-dimethylformamide, is used in an amount that is in the range between 0.01 and 0.5 equivalents, 0.05 and 0.2 equivalents, or 0.05 and 0.1 equivalents, based on the amount of compound II.

The process of the present invention is conducted either in an auxiliary solvent, or in the absence of an auxiliary solvent. The term "auxiliary solvent" herein refers to an inert aprotic organic solvent, which acts merely as a solvent and is not consumed in the course of the reaction. For the avoidance of doubt an auxiliary solvent is not identical with the reactants such as compounds II, the chlorinating agent, *N,N-*dimethylformamide or *N,N*-dimethylacetamide. Suitable auxiliary solvents are, for example, aliphatic, cycloaliphatic and aromatic hydrocarbons (non-limiting examples are: pentane, hexane, petroleum ether, cyclohexane, methylcyclohexane, benzene, toluene, xylene), aliphatic halogen-hydrocarbons (non-limiting examples are: methylene chloride, chloroform, di- and tetrachloroethane), nitriles (non-limiting examples are: acetonitrile, propionitrile, benzonitrile), ethers (non-limiting examples are: diethylether, dibutylether, tert-butylmethylether, ethylene glycol dimethyl ether, ethylene glycol, diethyl ether, diethylene glycol dimethyl ether, dioxane, diethylene, glycol monomethyl- or monoethyl ether), and sulphoxides and sulphones (non-limiting examples are: dimethyl sulfoxide, dimethyl sulfone, tetramethylene sulfoxide, tetramethylene sulfone).

Preferred auxiliary solvents are dioxane, *tert*-butyl methyl ether, di-*iso*-propyl ether, benzene, toluene, xylene, mesitylene, chlorobenzene, *n*-hexane, cyclohexane, dichloromethane, chloroform, tetrachloromethane, dichloroethane, or mixtures thereof.

In another preferred aspect the process is conducted in the absence of an auxiliary solvent.

In one embodiment the process is conducted at a concentration of at least 10% by weight of compound II, based on the total reaction medium. In another preferred embodiment the oxidation process is conducted at a concentration of at least 15% by weight of compound II, based on the total reaction medium.

The reaction mixture in these processes, with or without an auxiliary solvent, is usually heated to reflux temperature or to a temperature that is within the range between the reflux temperature and a temperature that lies 50°C below the reflux temperature; preferably the reaction mixture is heated to a temperature that is within the range between the reflux temperature and a temperature that lies 30°C below the reflux temperature; more preferably the reaction mixture is heated to a temperature that is within the range between the reflux temperature and a temperature that lies 10°C below the reflux temperature. In a particularly preferred embodiment the process of the present invention the reaction mixture is heated at reflux.

The reaction is carried out at pressures within a range between 100 kPa (1 bar) and 500 kPa, preferably between 100 kPa and 300 kPa.

The reaction is generally carried out within 1 to 12 hours; preferably within 1 to 8 hours; more preferably within 1 to 6 hours. Even more preferred is a reaction time within 1 to 4 hours.

In one aspect of the present invention the variable n is 1 and R is fluorine.

In a preferred embodiment the variable n is 0.

In a preferred embodiment (embodiment E.1) of the present invention the chlorinating reagent is phosphoryl trichloride.

Embodiment E.2: is based on embodiment E.1, wherein the amount of the chlorinating agent is between 2 and 15 equivalents.

Embodiment E.3: is based on embodiment E.2, wherein the auxiliary solvent is dioxane, *tert-*butyl methyl ether, di-*iso-*propyl ether, benzene, toluene, xylene, mesitylene, chlorobenzene, *n*-hexane, cyclohexane, dichloromethane, chloroform, tetrachloromethane, dichloroethane, or mixtures thereof; or in the absence of an auxiliary solvent.

Embodiment E.4: is based on embodiment E.3, wherein the reaction mixture is heated to a temperature that is within the range between the reflux temperature and a temperature that lies 50°C below the reflux temperature.

Embodiment E.5: is based on embodiment E.3, wherein the reaction mixture is heated to a temperature that is within the range between the reflux temperature and a temperature that lies 30°C below the reflux temperature.

Embodiment E.6: is based on embodiment E.3, wherein the reaction mixture is heated to a temperature that is within the range between the reflux temperature and a temperature that lies 10°C below the reflux temperature.

Embodiment E.7: is based on embodiment E.4, wherein the pressure is within a range between 100 kPa and 500 kPa.

Embodiment E.8: is based on embodiment E.5, wherein the pressure is within a range between 100 kPa and 500 kPa.

Embodiment E.9: is based on embodiment E.6, wherein the pressure is within a range between 100 kPa and 500 kPa.

Embodiment E.10: is based on embodiment E.4, wherein the pressure is within a range between 100 kPa and 300 kPa.

Embodiment E.11: is based on embodiment E.5, wherein the pressure is within a range between 100 kPa and 300 kPa.

Embodiment E.12: is based on embodiment E.6, wherein the pressure is within a range between 100 kPa and 300 kPa.

Compounds of formula II can be obtained in a two-step process, which involves reacting a compound of formula Ila 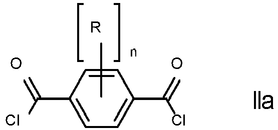 in the presence of water as described in Journal of Organic Chemistry 1953, 18, 1664-1669 to give a compound of formula Ilb 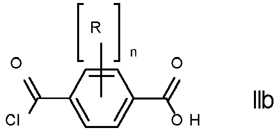 which is further reacted with ammonia as described in Journal of Organic Chemistry 2016, 81(5), 2166-2173 to give a compound of formula II, whereas the variables n and R in compounds IIa and IIb are as defined for compounds of formulae I and II herein.

The dichlorides Ila are either commercially available or they can be prepared from commercially available starting materials using synthetic procedures that are well known to the skilled person in the art.

In a further embodiment the present invention relates to a process comprising the step of reacting the compound of formula I, wherein the variable n is 0, with an amine of formula III,

R¹-NH-R² III

wherein
- R¹: is C₁-C₆-alkyl, C₁-C₆-alkoxy, C₃-C₁₁-cycloalkyl, C₃-C₈-cycloalkenyl, C₂-C₆-alkenyl, C₂-C₆-alkynyl, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, C₁-C₆-alkylamino, diC₁-C₆-alkylamino, -C(=O)-C₁-C₆-alkyl, -C(=O)-O-C₁-C₆-alkyl, phenyl-C₁-C₄-alkyl, phenyl-C₁-C₄-alkenyl, phenyl-C₁-C₄-alkynyl, heteroaryl-C₁-C₄-alkyl, phenyl, naphthyl, or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein the heteroaryl group in the group heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O, and S as ring member atoms with the provision that the heterocycle cannot contain 2 contiguous atoms selected from O and S; and wherein any of the above-mentioned aliphatic or cyclic groups are unsubstituted or substituted with 1, 2, 3, or up to the maximum possible number of identical or different groups R^{1a}; or
- R¹ and R²,: together with the nitrogen atom to which they are attached, form a saturated or partially unsaturated mono- or bicyclic 3- to 10-membered heterocycle, wherein the heterocycle includes beside one nitrogen atom and one or more carbon atoms no further heteroatoms or 1, 2 or 3 further heteroatoms independently selected from N, O, and S as ring member atoms with the provision that the heterocycle cannot contain 2 contiguous atoms selected from O and S; and wherein the heterocycle is unsubstituted or substituted with 1, 2, 3, 4, or up to the maximum possible number of identical or different groups R^{1a}; wherein
- R^{1a}: is halogen, oxo, cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂-C₁-C₄-alkyl, -(C=O)-C₁-C₄-alkyl, C(=O)-C₁-C₄-alkyl, C₁-C₆-alkylsulfonyl, hydroxyC₁-C₄-alkyl, -C(=O)-NH₂, -C(=O)-NH(C₁-C₄-alkyl), C₁-C₄-alkylthio-C₁-C₄-alkyl, aminoC₁-C₄-alkyl, C₁-C₄-alkylamino-C₁-C₄-alkyl, diC₁-C₄-alkylamino-C₁-C₄-alkyl, aminocarbonyl-C₁-C₄-alkyl, or C₁-C₄-alkoxy-C₁-C₄-alkyl;
- R²: is hydrogen, C₁-C₆-alkyl, C₂-C₆-alkenyl, C₂-C₆-alkynyl, C₁-C₆-alkoxy, C₃-C₁₁-cycloalkyl, -C(=O)H, -C(=O)-C₁-C₆-alkyl, -C(=O)-C₃-C₁₁-cycloalkyl, or-C(=O)-O-C₁-C₆-alkyl; and wherein any of the aliphatic or cyclic groups in R² are unsubstituted or substituted with 1, 2, 3, or up to the maximum possible number of identical or different radicals selected from the group consisting of halogen, hydroxy, oxo, cyano, C₁-C₆-alkyl, C₁-C₆-alkoxy, and C₃-C₁₁-cycloalkyl;

to obtain a compound of formula IV

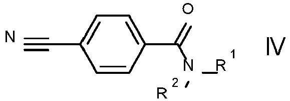

Analogous transformations are described in WO 2013/008162 A1, WO 2015/185485 A1, or WO 2017/211652 A1 and the references cited therein.

The amines of formula III are either commercially available or can be prepared, for example, according to R. C. Larock, Comprehensive Organic Transformations, Verlag Wiley-VCH, 2nd Edition 1999, pages 1929 ff..

In a further embodiment the present invention relates to a process comprising the step of reacting the compound of formula IV with hydroxylamine or its hydrochloride salt, in the presence of a base, preferably triethylamine, sodium hydroxide or sodium methylate, in a suitable solvent, such as methanol, ethanol or water, or a mixture of these solvents, at a temperature between 0°C and 100°C to obtain a compound of formula Va 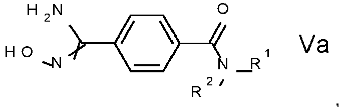 which is further reacted with an activated derivative of trifluoroacetic acid, for example ethyl trifluoroacetate, trifluoroacetic anhydride or trifluoroacetic chloride, to obtain a compound of formula V

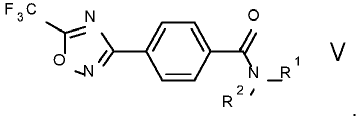

For related examples see Kitamura, S. et al Chem. Pharm. Bull. 2001, 49, 268 or WO 2013/008162 A1 or WO 2015/185485 A1.

In another embodiment, the compound of formula V is reacted with a suitable thionylating reagent to obtain a compound of formula VI 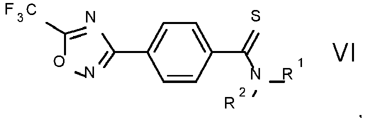 as described in WO 2019/020451 A1 and WO 2017/211649 A1 and the references cited therein.

In a preferred embodiment the variables R¹ and R² in compounds of formula III, IV, V and VI have the following meaning:
- R¹: is methyl, ethyl, *n*-propyl, *iso*-propyl, *n*-butyl, *sec*-butyl, *iso*-butyl, cyclopropyl, 2-methoxyiminoethyl, bicyclo[1.1.1]pentan-1-yl, or phenyl; and wherein the phenyl group is unsubstituted or substituted with 1, 2, 3 or up to the maximum possible number of identical or different radicals selected from the group consisting of fluorine, chlorine, cyano, OH, NH₂, methyl, ethyl, methoxy, trifluoromethyl, trifluoromethoxy, difluoromethyl, difluoromethoxy, and cyclopropyl; and
- R²: is hydrogen, methyl, or ethyl.

In another preferred embodiment the variables R¹ and R² in compounds of formula III, IV, V and VI have the following meaning:
- R¹: is methyl, 2-methoxyiminoethyl, bicyclo[1.1.1]pentan-1-yl, 2-fluoro-phenyl, 4-fluorophenyl, or 2,4-difluorophenyl; in particular methyl or 2-fluoro-phenyl; and
- R²: is hydrogen.

In the definitions of the variables given above, collective terms are used which are generally representative for the substituents in question.

The term "Cₙ-Cₘ" indicates the number of carbon atoms possible in each case in the substituent or substituent moiety in question.

The term "halogen" refers to fluorine, chlorine, bromine and iodine.

The term "oxo" refers to an oxygen atom =O, which is bound to a carbon atom or sulfur atom, thus forming, for example, a ketonyl -C(=O)- or sulfinyl -S(=O)- group.

The term "C₁-C₆-alkyl" refers to a straight-chained or branched saturated hydrocarbon group having 1 to 6 carbon atoms, for example methyl, ethyl, propyl, 1-methylethyl, butyl, 1-methylpropyl, 2-methylpropyl, and 1,1-dimethylethyl.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₁-C₆-haloalkyl" refers to a straight-chained or branched alkyl group having 1 to 6 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "C₁-C₆-alkoxy" refers to a straight-chain or branched alkyl group having 1 to 6 carbon atoms (as defined above) which is bonded via an oxygen, at any position in the alkyl group, for example methoxy, ethoxy, n-propoxy, 1-methylethoxy, butoxy, 1-methylpropoxy, 2-methylpropoxy or 1,1-dimethylethoxy.

The term "C₁-C₆-haloalkoxy" refers to a C₁-C₆-alkoxy group as defined above, wherein some or all of the hydrogen atoms may be replaced by halogen atoms as mentioned above, for example, OCH₂F, OCHF₂, OCF₃, OCH₂Cl, OCHCl₂, OCClₐ, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy.

The terms "phenyl-C₁-C₄-alkyl or heteroaryl-C₁-C₄-alkyl" refer to alkyl having 1 to 4 carbon atoms (as defined above), wherein one hydrogen atom of the alkyl radical is replaced by a phenyl or hetereoaryl radical respectively.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₄-alkylthio-C₁-C₄-alkyl" refers to alkyl having 1 to 4 carbon atoms (as defined above), wherein one hydrogen atom of the alkyl radical is replaced by a C₁-C₄-alkylthio group.

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₄-alkoxyimino" refers to a divalent imino radical (C₁-C₄-alkyl-O-N=) carrying one C₁-C₄-alkoxy group as substituent, e.g. methylimino, ethylimino, propylimino, 1-methylethyl-imino, butylimino, 1-methylpropylimino, 2-methylpropylimino, 1,1-dimethylethylimino and the like.

The term "C₁-C₆-alkoxyimino-C₁-C₄-alkyl" refers to alkyl having 1 to 4 carbon atoms, wherein two hydrogen atoms of one carbon atom of the alkyl radical are replaced by a divalent C₁-C₆-alkoxyimino radical (C₁-C₆-alkyl-O-N=) as defined above.

The term "C₂-C₆-alkenyloxyimino-C₁-C₄-alkyl" refers to alkyl having 1 to 4 carbon atoms, wherein two hydrogen atoms of one carbon atom of the alkyl radical are replaced by a divalent C₂-C₆-alkenyloxyimino radical (C₂-C₆-alkenyl-O-N=).

The term "C₂-C₆-alkynyloxyimino-C₁-C₄-alkyl" refers to alkyl having 1 to 4 carbon atoms, wherein two hydrogen atoms of one carbon atom of the alkyl radical are replaced by a divalent C₂-C₆-alkynyloxyimino radical (C₂-C₆-alkynyl-O-N=).

The term "hydroxyC₁-C₄-alkyl" refers to alkyl having 1 to 4 carbon atoms, wherein one hydrogen atom of the alkyl radical is replaced by a OH group.

The term "aminoC₁-C₄-alkyl" refers to alkyl having 1 to 4 carbon atoms, wherein one hydrogen atom of the alkyl radical is replaced by a NH₂ group.

The term "C₁-C₆-alkylamino" refers to an amino group, which is substituted with one residue independently selected from the group that is defined by the term C₁-C₆-alkyl. Likewise, the term "diC₁-C₆-alkylamino" refers to an amino group, which is substituted with two residues independently selected from the group that is defined by the term C₁-C₆-alkyl.

The term "C₁-C₄-alkylamino-C₁-C₄-alkyl" refers to refers to alkyl having 1 to 4 carbon atoms (as defined above), wherein one hydrogen atom of the alkyl radical is replaced by a C₁-C₄-alkyl-NH-group which is bound through the nitrogen. Likewise, the term "diC₁-C₄-alkylamino-C₁-C₄-alkyl" refers to refers to alkyl having 1 to 4 carbon atoms (as defined above), wherein one hydrogen atom of the alkyl radical is replaced by a (C₁-C₄-alkyl)₂N- group which is bound through the nitrogen.

The term "aminocarbonyl-C₁-C₄-alkyl" refers to alkyl having 1 to 4 carbon atoms, wherein one hydrogen atom of the alkyl radical is replaced by a -(C=O)-NH₂ group.

The term "C₃-C₁₁-cycloalkyl" refers to a monocyclic, bicyclic or tricyclic saturated univalent hydrocarbon radical having 3 to 11 carbon ring members that is connected through one of the ring carbon atoms by substitution of one hydrogen atom, such as cyclopropyl (C₃H₅), cyclobutyl, cyclopentyl, cyclohexyl, cycloheptyl, cyclooctyl, bicyclo[1.1.0]butyl, bicyclo[2.1.0]pentyl, bicyclo[1.1.1]pentyl, bicyclo[3.1.0]hexyl, bicyclo[2.1.1]hexyl, norcaranyl (bicyclo[4.1.0]heptyl) and norbornyl (bicyclo[2.2.1]heptyl).

The terms "-C(=O)-C₁-C₆-alkyl", "-C(=O)-O-C₁-C₆-alkyl" and "-C(=O)-C₃-C₁₁-cycloalkyl" refer to aliphatic radicals which are attached through the carbon atom of the -C(=O)- group.

The term "aliphatic" refers to compounds or radicals composed of carbon and hydrogen and which are non-aromatic compounds. An "alicyclic" compound or radical is an organic compound that is both aliphatic and cyclic. They contain one or more all-carbon rings which may be either saturated or unsaturated, but do not have aromatic character.

The terms "cyclic moiety" or "cyclic group" refer to a radical which is an alicyclic ring or an aromatic ring, such as, for example, phenyl or heteroaryl.

The term "and wherein any of the aliphatic or cyclic groups are unsubstituted or substituted with..." refers to aliphatic groups, cyclic groups and groups, which contain an aliphatic and a cyclic moiety in one group, such as in, for example, C₃-C₈-cycloalkyl-C₁-C₄-alkyl; therefore a group which contains an aliphatic and a cyclic moiety both of these moieties may be substituted or unsubstituted independently of each other.

The term "phenyl" refers to an aromatic ring systems incuding six carbon atoms (commonly referred to as benzene ring.

The term "heteroaryl" refers to aromatic monocyclic or polycyclic ring systems incuding besides carbon atoms, 1, 2, 3 or 4 heteroatoms independently selected from the group consisting of N, O and S.

The term "saturated 3- to 7-membered carbocycle" is to be understood as meaning monocyclic saturated carbocycles having 3, 4 or 5 carbon ring members. Examples include cyclopropyl, cyclopentyl, cyclohexyl, cycloheptyl, and the like.

The term "3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms", is to be understood as meaning both, aromatic mono- and bicyclic heteroaromatic ring systems, and also saturated and partially unsaturated heterocycles, for example:
a 3- or 4-membered saturated heterocycle which contains 1 or 2 heteroatoms from the group consisting of N, O and S as ring members such as oxirane, aziridine, thiirane, oxetane, azetidine, thiethane, [1,2]dioxetane, [1,2]dithietane, [1,2]diazetidine;
and a 5- or 6-membered saturated or partially unsaturated heterocycle which contains 1, 2 or 3 heteroatoms from the group consisting of N, O and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetrahydro[2H]azepin-2-,-3-,-4-,-5-,-6- or-7-yl, 2,3,4,7-tetrahydro[1H]azepin-1-,-2-,-3-,-4-,-5-,-6-or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals.

The term "5- or 6-membered heteroaryl" or the term "5- or 6-membered aromatic heterocycle" refer to aromatic ring systems incuding besides carbon atoms, 1, 2, 3 or 4 heteroatoms independently selected from the group consisting of N, O and S, for example, a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or
a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

### Working Examples

The present invention is further illustrated by means of the following working examples.

### HPLC Methodology

HPLC device: Agilent 1100 Series; column: Agilent Zorbax Eclipse XDB-C18 1,8 µm 50*4,6mm von Agilent, Column Flow: 1,3 mL/min, time: 10 min, pressure: 23000 kPa; temperature: 20°C; wavelength 195 nm; injector volume: 1 uL; retention time of the respective products is based on reference material and given below. Eluent: A: Water with 0,1 vol% H₃PO₄; B: Acetonitrile

| Time (min) | %B | Rate (mL/min) |
|---|---|---|
| 0,0 | 0 | 1,3 |
| 2,0 | 0 | 1,3 |
| 5,0 | 80 | 1,3 |
| 6,0 | 100 | 1,3 |
| 8,0 | 100 | 1,3 |
| 8,1 | 0 | 1,3 |

### Example 1) Preparation of 4-carbamoylbenzoic acid

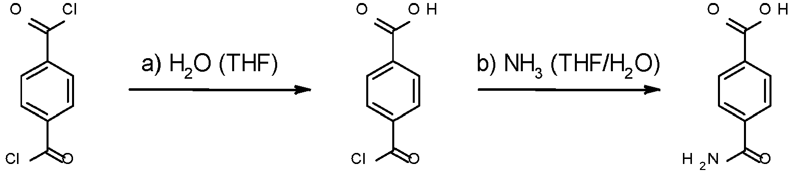

Step a): a three necked flask was charged with tetrahydrofuran (10 mL) and terephthalic acid dichloride (2.4 g, 11.8 mmol, commercially available). A solution of water (224 mg, 12.4 mmol, 1.05 eq.) in tetrahydrofuran (10 mL) was added dropwise and the resulting mixture was stirred at ambient temperature for 2 hours (for reaction control: an aliquot is quenched with methanol and the resulting mixture is analyzed via HPLC).

Step b): the crude product was transferred to a dropping funnel and added dropwise to a mixture of ammonia (33% w/w in water, 3.8 g, 35.4 mmol) and tetrahydrofuran (12 mL) over a period of 25 minutes. The resulting suspension was stirred for 1 hour at ambient temperature (for reaction control: an aliquot is quenched with methanol and the resulting mixture is analyzed via HPLC), water was added and the mixture was stirred for additional 30 minutes. An aqueous solution of hydrogen chloride (37% w/w in water) was added to adjust the pH value to 1 and stirring was continued for 30 minutes. The solids were collected by filtration, washed with water (2x 10 mL) and dried under vacuum at 40°C. HPLC analysis showed 68 area% of 4-carbamoylbenzoic acid (retention time 4.90 min) along with 19% diamide, 11% diacid, 2% others.

Similar experiments were conducted with varying water amounts:
The use of 0.9 eq. water with otherwise unchanged conditions delivers a mixture of:
   monoacid - 71 area% (retention time 4.90 min), diacid - 9 area%, diamide - 18 area%.
The use of 1.2 eq. water with otherwise unchanged conditions delivers a mixture of:
   monoacid - 71 area% (retention time 4.90 min), diacid - 18 area%, diamide - 8 area%.

### Example 2) Preparation of 4-cyanobenzoyl chloride

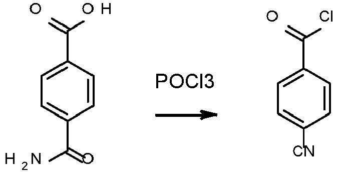

A mixture (8 g) obtained by the procedure of Example 1) containing the mono-acid (72.1 area%), the di-acid (4.9 area%) and the diamide (13.8 area%) was stirred with phosphoryl trichloride (52 g, 339 mmol) for 1 hour at an internal temperature of 80°C. During this time the solids dissolve completely in the chlorinating reagent. An aliquot is carefully quenched with warm water and the solids dissolved with acetonitrile. HPLC analysis shows the desired product as acid (69.1 area% - retention time 6,38 min). Excess phosphoryl trichloride was removed by distillation and careful quench of the residue with warm water. Filtration of the solid yielded a mixture of 4-cyanobenzoic acid, the terephthalic dinitrile and terephthalic acid. Alternatively, removal of the phosphoryl trichloride by distillation may be followed by vacuum distillation of the residue to isolate pure acid chloride.

## Claims

1. A process for the preparation of 4-cyanobenzoyl chlorides of formula I, 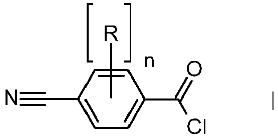 wherein
R is halogen, C₁-C₄-alkyl, C₁-C₄-haloalkyl, C₁-C₄-alkoxy, or C₁-C₄-haloalkoxy;
n is 0, 1 or 2;
the process comprising reacting a compound of formula II, 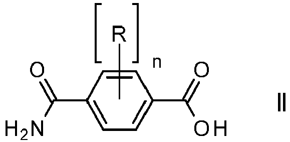 wherein the variable R is as defined above for compounds of formula I, with a chlorinating agent.

2. The process according to claim 1, wherein the chlorinating agent is selected from phosphoryl trichloride, trichlorophosphane, pentachlorophosphane, thionyl chloride, phosgene, diphosgene, triphosgene, and oxalyl chloride.

3. The process according to claim 1, wherein the chlorinating agent is phosphoryl trichloride.

4. The process according to any one of claims 1 to 3, wherein the amount of chlorinating agent is between 2 and 15 equivalents based on the amount of compound of formula II.

5. The process according to any one of claims 1 to 4, wherein the process is conducted in an auxiliary solvent or in the absence of an auxiliary solvent.

6. The process according to claim 5, wherein the auxiliary solvent is dioxane, *tert*-butyl methyl ether, di-*iso*-propyl ether, benzene, toluene, xylene, mesitylene, chlorobenzene, *n*-hexane, cyclohexane, dichloromethane, chloroform, tetrachloromethane, dichloroethane, or mixtures thereof.

7. The process according to any one of claims 1 to 4, wherein the reaction is conducted in the absence of a solvent.

8. The process according to any one of claims 1 to 7, wherein the reaction is conducted in the presence of substoichiometric amounts of *N*,*N*-dimethylformamide or *N,N-*dimethylacetamide based on the amount of compound II.

9. The process according to any one of claims 1 to 8, wherein the reaction mixture is heated to reflux temperature or to a temperature that is within the range between the reflux temperature and a temperature that lies 50°C below the reflux temperature.

10. The process according to any one of claims 1 to 9, wherein the pressure is within the range between 100 kPa and 500 kPa.

11. The process according to any one of claims 1 to 10, wherein the variable n is 1 and R in compounds of formulae I and II is fluorine.

12. The process according to any one of claims 1 to 10, wherein the variable n is 0 in compounds of formulae I and II.

13. The process according to any one of claims 1 to 12, further comprising reacting the compound of formula Ilb, wherein the variables n and R are as defined for compounds of formulae I and II, 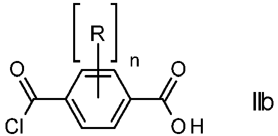 to give a compound of formula II.

14. The process according to claim 13, further comprising reacting the compound of formula Ila, wherein the variables n and R are as defined for compounds of formulae I and II, in the presence of water 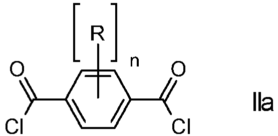 to give a compound of formula Ilb.

15. The process according to any one of claims 12 to 14, further comprising the step of reacting the compound of formula I with an amine of formula III,
R¹-NH-R² III
wherein
R¹ is C₁-C₆-alkyl, C₁-C₆-alkoxy, C₃-C₁₁-cycloalkyl, C₃-C₈-cycloalkenyl, C₂-C₆-alkenyl, C₂-C₆-alkynyl, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, C₁-C₆-alkylamino, diC₁-C₆-alkylamino, -C(=O)-C₁-C₆-alkyl, -C(=O)-O-C₁-C₆-alkyl, phenyl-C₁-C₄-alkyl, phenyl-C₁-C₄-alkenyl, phenyl-C₁-C₄-alkynyl, heteroaryl-C₁-C₄-alkyl, phenyl, naphthyl, or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein the heteroaryl group in the group heteroarylC₁-C₄-alkyl is a 5- or 6-membered aromatic heterocycle, wherein the ring member atoms of the heterocyclic ring include besides carbon atoms 1, 2, 3 or 4 heteroatoms selected from N, O, and S as ring member atoms with the provision that the heterocycle cannot contain 2 contiguous atoms selected from O and S; and wherein any of the above-mentioned aliphatic or cyclic groups are unsubstituted or substituted with 1, 2, 3, or up to the maximum possible number of identical or different groups R^{1a}; or
R¹ and R², together with the nitrogen atom to which they are attached, form a saturated or partially unsaturated mono- or bicyclic 3- to 10-membered heterocycle, wherein the heterocycle includes beside one nitrogen atom and one or more carbon atoms no further heteroatoms or 1, 2 or 3 further heteroatoms independently selected from N, O, and S as ring member atoms with the provision that the heterocycle cannot contain 2 contiguous atoms selected from O and S; and wherein the heterocycle is unsubstituted or substituted with 1, 2, 3, 4, or up to the maximum possible number of identical or different groups R^{1a}; wherein
R^{1a} is halogen, oxo, cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂-C₁-C₄-alkyl, -(C=O)-C₁-C₄-alkyl, C(=O)-C₁-C₄-alkyl, C₁-C₆-alkylsulfonyl, hydroxyC₁-C₄-alkyl, -C(=O)-NH₂, -C(=O)-NH(C₁-C₄-alkyl), C₁-C₄-alkylthio-C₁-C₄-alkyl, aminoC₁-C₄-alkyl, C₁-C₄-alkylamino-C₁-C₄-alkyl, diC₁-C₄-alkylamino-C₁-C₄-alkyl, aminocarbonyl-C₁-C₄-alkyl, or C₁-C₄-alkoxy-C₁-C₄-alkyl;
R² is hydrogen, C₁-C₆-alkyl, C₂-C₆-alkenyl, C₂-C₆-alkynyl, C₁-C₆-alkoxy, C₃-C₁₁-cycloalkyl, -C(=O)H, -C(=O)-C₁-C₆-alkyl, -C(=O)-C₃-C₁₁-cycloalkyl, or -C(=O)-O-C₁-C₆-alkyl; and wherein any of the aliphatic or cyclic groups in R² are unsubstituted or substituted with 1, 2, 3, or up to the maximum possible number of identical or different radicals selected from the group consisting of halogen, hydroxy, oxo, cyano, C₁-C₆-alkyl, C₁-C₆-alkoxy, and C₃-C₁₁-cycloalkyl;
to obtain a compound of formula IV

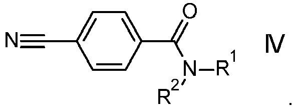

16. The process according to claim 15, further comprising reacting the compound of formula IV to obtain a compound of formula V

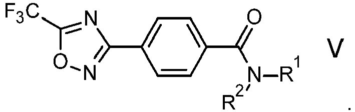

17. The process according to claim 16, further comprising the step of reacting the compound of formula V to obtain a compound of formula VI

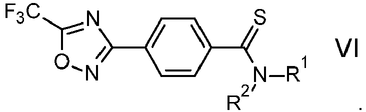

18. The process according to any one of claims 14 to 17, wherein in compounds of formula III, IV, V and VI
R¹ is methyl, 2-methoxyiminoethyl, bicyclo[1.1.1]pentan-1-yl, 2-fluoro-phenyl, 4-fluorophenyl, or 2,4-difluorophenyl; in particular methyl or 2-fluoro-phenyl; and
R² is hydrogen.

## Patentansprüche

1. Verfahren zur Herstellung von 4-Cyanobenzoylchloriden der Formel I, 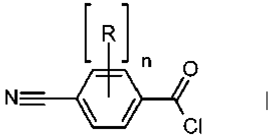 wobei
R für Halogen, C₁-C₄-Alkyl, C₁-C₄-Halogenalkyl, C₁-C₄-Alkoxy oder C₁-C₄-Halogenalkoxy steht;
n für 0, 1 oder 2 steht;
wobei das Verfahren das Umsetzen einer Verbindung der Formel II, 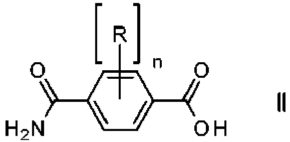
wobei die Variable R wie oben für Verbindungen der Formel I definiert ist, mit einem Chlorierungsmittel umfasst.

2. Verfahren nach Anspruch 1, wobei das Chlorierungsmittel aus Phosphoryltrichlorid, Trichlorphosphan, Pentachlorphosphan, Thionylchlorid, Phosgen, Diphosgen, Triphosgen und Oxalylchlorid ausgewählt wird.

3. Verfahren nach Anspruch 1, wobei es sich bei dem Chlorierungsmittel um Phosphoryltrichlorid handelt.

4. Verfahren nach einem der Ansprüche 1 bis 3, wobei die Menge an Chlorierungsmittel zwischen 2 und 15 Äquivalenten, bezogen auf die Menge an Verbindung der Formel II, liegt.

5. Verfahren nach einem der Ansprüche 1 bis 4, wobei das Verfahren in einem Hilfslösungsmittel oder in Abwesenheit eines Hilfslösungsmittels durchgeführt wird.

6. Verfahren nach Anspruch 5, wobei es sich bei dem Hilfslösungsmittel um Dioxan, tert-Butylmethylether, Di-iso-propylether, Benzol, Toluol, Xylol, Mesitylen, Chlorbenzol, n-Hexan, Cyclohexan, Dichlormethan, Chloroform, Tetrachlorethan, Dichlorethan oder Mischungen davon handelt.

7. Verfahren nach einem der Ansprüche 1 bis 4, wobei die Umsetzung in Abwesenheit eines Lösungsmittels durchgeführt wird.

8. Verfahren nach einem der Ansprüche 1 bis 7, wobei die Umsetzung in Gegenwart von unterstöchiometrischen Mengen an *N,N-*Dimethylformamid oder *N,N-*Dimethylacetamid, bezogen auf die Menge an Verbindung II, durchgeführt wird.

9. Verfahren nach einem der Ansprüche 1 bis 8, wobei die Reaktionsmischung auf Rückflusstemperatur oder auf eine Temperatur, die im Bereich zwischen der Rückflusstemperatur und einer 50 °C unter der Rückflusstemperatur liegenden Temperatur liegt, erhitzt wird.

10. Verfahren nach einem der Ansprüche 1 bis 9, wobei der Druck im Bereich zwischen 100 kPa und 500 kPa liegt.

11. Verfahren nach einem der Ansprüche 1 bis 10, wobei die Variable n für 1 steht und R in Verbindungen der Formeln I und II für Fluor steht.

12. Verfahren nach einem der Ansprüche 1 bis 10, wobei die Variable n in Verbindungen der Formeln I und II für 0 steht.

13. Verfahren nach einem der Ansprüche 1 bis 12, ferner umfassend das Umsetzen der Verbindung der Formel IIb, wobei die Variablen n und R wie für Verbindungen der Formel I und II definiert sind, 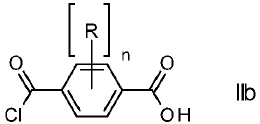 zu einer Verbindung der Formel II.

14. Verfahren nach Anspruch 13, ferner umfassend das Umsetzen der Verbindung der Formel IIa, wobei die Variablen n und R wie für Verbindungen der Formel I und II definiert sind, in Gegenwart von Wasser 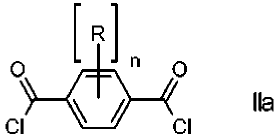 zu einer Verbindung der Formel IIb.

15. Verfahren nach einem der Ansprüche 12 bis 14, ferner umfassend den Schritt des Umsetzens der Verbindung der Formel I mit einem Amin der Formel III,
R¹-NH-R² III
wobei
R¹ für C₁-C₆-Alkyl, C₁-C₆-Alkoxy, C₃-C₁₁-Cycloalkyl, C₃-C₈-Cycloalkenyl, C₂-C₆-Alkenyl, C₂-C₆-Alkinyl, C₁-C₆-Alkoxyimino-C₁-C₄-alkyl, C₂-C₆-Alkenyloxyimino-C₁-C₄-alkyl, C₂-C₆-Alkinyloxyimino-C₁-C₄-alkyl, C₁-C₆-Alkylamino, Di-C₁-C₆-alkylamino, -C(=O)-C₁-C₆-Alkyl, - C(=O)-O-C₁-C₆-Alkyl, Phenyl-C₁-C₄-alkyl, Phenyl-C₁-C₄-alkenyl, Phenyl-C₁-C₄-alkinyl, Heteroaryl-C₁-C₄-alkyl, Phenyl, Naphthyl oder einen 3- bis 10-gliedrigen gesättigten, teilweise ungesättigten oder aromatischen mono- oder bicyclischen Heterocyclus steht, wobei die Ringgliedatome des mono- oder bicyclischen Heterocyclus neben Kohlenstoffatomen ferner 1, 2, 3 oder 4 Heteroatome, die aus N, O und S ausgewählt sind, als Ringgliedatome einschließen, mit der Maßgabe, dass der Heterocyclus keine 2 benachbarten Atome, die aus O und S ausgewählt sind, enthalten kann; und wobei es sich bei der Heteroarylgruppe in der Gruppe Heteroaryl-C₁-C₄-alkyl um einen 5- oder 6-gliedrigen aromatischen Heterocyclus handelt, wobei die Ringgliedatome des heterocyclischen Rings neben Kohlenstoffatomen 1, 2, 3 oder 4 Heteroatome, die aus N, O und S ausgewählt sind, als Ringgliedatome einschließen, mit der Maßgabe, dass der Heterocyclus keine 2 benachbarten Atome, die aus O und S ausgewählt sind, enthalten kann; und wobei jede der oben aufgeführten aliphatischen oder cyclischen Gruppen unsubstituiert oder durch 1, 2, 3 oder bis zur maximal möglichen Anzahl gleiche oder verschiedene Gruppen R^{1a} substituiert ist; oder
R¹ und R² zusammen mit dem Stickstoffatom, an das sie gebunden sind, einen gesättigten oder teilweise ungesättigten mono- oder bicyclischen 3- bis 10-gliedrigen Heterocyclus bilden, wobei der Heterocyclus neben einem Stickstoffatom und einem oder mehreren Kohlenstoffatomen keine weiteren Heteroatome oder 1, 2 oder 3 weitere Heteroatome, die unabhängig aus N, O und S ausgewählt sind, als Ringgliedatome einschließt, mit der Maßgabe, dass der Heterocyclus keine 2 benachbarten Atome, die aus O und S ausgewählt sind, enthalten kann; und wobei der Heterocyclus unsubstituiert oder durch 1, 2, 3, 4 oder bis zur maximal möglichen Anzahl gleiche oder verschiedene Gruppen R^{1a} substituiert ist; wobei
R^{1a} für Halogen, Oxo, Cyano, NO₂, OH, SH, NH₂, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, - NHSO₂-C₁-C₄-Alkyl, -(C=O)-C₁-C₄-Alkyl, C(=O)-C₁-C₄-Alkyl, C₁-C₆-Alkylsulfonyl, Hydroxy-C₁-C₄-alkyl, -C(=O)-NH₂, - C(=O)-NH(C₁-C₄-Alkyl), C₁-C₄-Alkylthio-C₁-C₄-alkyl, Amino-C₁-C₄-alkyl, C₁-C₄-Alkylamino-C₁-C₄-alkyl, Di-C₁-C₄-alkylamino-C₁-C₄-alkyl, Aminocarbonyl-C₁-C₄-alkyl oder C₁-C₄-Alkoxy-C₁-C₄-alkyl steht;
R² für Wasserstoff, C₁-C₆-Alkyl, C₂-C₆-Alkenyl, C₂-C₆-Alkinyl, C₁-C₆-Alkoxy, C₃-C₁₁-Cycloalkyl, -C(=O)H, -C(=O)-C₁-C₆-Alkyl, -C(=O)-C₃-C₁₁-Cycloalkyl oder -C(=O)-O-C₁-C₆-Alkyl steht und wobei jede der aliphatischen oder cyclischen Gruppen in R² unsubstituiert oder durch 1, 2, 3 oder bis zur maximal möglichen Anzahl gleiche oder verschiedene Reste, die aus der Gruppe bestehend aus Halogen, Hydroxy, Oxo, Cyano, C₁-C₆-Alkyl, C₁-C₆-Alkoxy und C₃-C₁₁-Cycloalkyl ausgewählt sind, substituiert ist; zu einer Verbindung der Formel IV

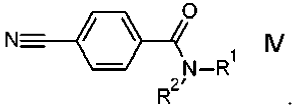

16. Verfahren nach Anspruch 15, ferner umfassend das Umsetzen der Verbindung der Formel IV zu einer Verbindung der Formel V

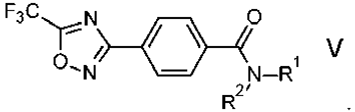

17. Verfahren nach Anspruch 16, ferner umfassend den Schritt des Umsetzens der Verbindung der Formel V zu einer Verbindung der Formel VI

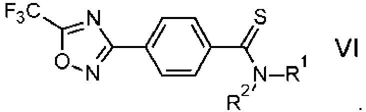

18. Verfahren nach einem der Ansprüche 14 bis 17, wobei in Verbindungen der Formel III, IV, V und VI
R¹ für Methyl, 2-Methoxyiminoethyl, Bicyclo[1.1.1]pentan-1-yl, 2-Fluorphenyl, 4-Fluorphenyl oder 2,4-Difluorphenyl; insbesondere Methyl oder 2-Fluorphenyl; steht und
R² für Wasserstoff steht.

## Revendications

1. Procédé de préparation de chlorures de 4-cyanobenzoyle de formule I, 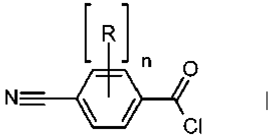
R étant halogène, C₁-C₄-alkyle, C₁-C₄-halogénoalkyle, C₁-C₄-alcoxy, ou C₁-C₄-halogénoalcoxy ;
n étant 0, 1 ou 2 ;
le procédé comprenant la mise en réaction d'un composé de formule II, 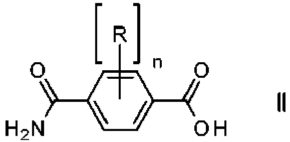
la variable R étant telle que définie ci-dessus pour des composés de formule I, avec un agent de chloration.

2. Procédé selon la revendication 1, l'agent de chloration étant choisi parmi le trichlorure de phosphoryle, le trichlorophosphane, le pentachlorophosphane, le chlorure de thionyle, le phosgène, le diphosgène, le triphosgène et le chlorure d'oxalyle.

3. Procédé selon la revendication 1, l'agent de chloration étant le trichlorure de phosphoryle.

4. Procédé selon l'une quelconque des revendications 1 à 3, la quantité d'agent de chloration étant comprise entre 2 et 15 équivalents sur la base de la quantité de composé de formule II.

5. Procédé selon l'une quelconque des revendications 1 à 4, le procédé étant conduit dans un solvant auxiliaire ou en l'absence d'un solvant auxiliaire.

6. Procédé selon la revendication 5, le solvant auxiliaire étant le dioxane, l'éther tert-butylméthylique, l'éther diisopropylique, le benzène, le toluène, un xylène, le mésitylène, le chlorobenzène, le n-hexane, le cyclohexane, le dichlorométhane, le chloroforme, le tétrachlorométhane, le dichloroéthane ou des mélanges correspondants.

7. Procédé selon l'une quelconque des revendications 1 à 4, la réaction étant conduite en l'absence d'un solvant.

8. Procédé selon l'une quelconque des revendications 1 à 7, la réaction étant conduite en présence de quantités sous-stœchiométriques de N,N-diméthylformamide ou N,N-diméthylacétamide sur la base de la quantité de composé II.

9. Procédé selon l'une quelconque des revendications 1 à 8, le mélange réactionnel étant chauffé jusqu'à la température de reflux ou jusqu'à une température comprise dans la plage entre la température de reflux et une température inférieure de 50 °C à la température de reflux.

10. Procédé selon l'une quelconque des revendications 1 à 9, la pression étant comprise dans la plage entre 100 kPa et 500 kPa.

11. Procédé selon l'une quelconque des revendications 1 à 10, la variable n étant 1 et R dans les composés des formules I et II étant fluor.

12. Procédé selon l'une quelconque des revendications 1 à 10, la variable n étant 0 dans les composés des formules I et II.

13. Procédé selon l'une quelconque des revendications 1 à 12, comprenant en outre la mise en réaction du composé de formule IIb, les variables n et R étant comme définies pour les composés des formules I et II, 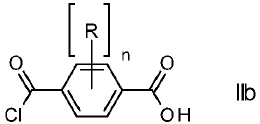 pour donner un composé de formule II.

14. Procédé selon la revendication 13, comprenant en outre la mise en réaction du composé de formule IIa, les variables n et R étant comme définies pour les composés des formules I et II, en présence d'eau 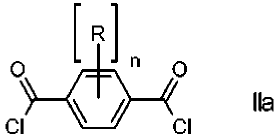 pour donner un composé de formule IIb.

15. Procédé selon l'une quelconque des revendications 12 à 14, comprenant en outre l'étape de mise en réaction du composé de formule I avec une amine de formule III,
R¹-NH-R² III
R¹ étant C₁-C₆-alkyle, C₁-C₆-alcoxy, C₃-C₁₁-cycloalkyle, C₃-C₈-cycloalcényle, C₂-C₆-alcényle, C₂-C₆-alcynyle, C₁-C₆-alcoxyimino-C₁-C₄-alkyle, C₂-C₆-alcényloxyimino-C₁-C₄-alkyle, C₂-C₆-alcynyloxyimino-C₁-C₄-alkyle, C₁-C₆-alkylamino, diC₁-C₆-alkylamino, -C(=O)-C₁-C₆-alkyle, - C(=O)-O-C₁-C₆-alkyle, phényl-C₁-C₄-alkyle, phényl-C₁-C₄-alcényle, phényl-C₁-C₄-alcynyle, hétéroaryl-C₁-C₄-alkyle, phényle, naphtyle, ou un hétérocycle monocyclique ou bicyclique saturé, partiellement insaturé ou aromatique à 3 à 10 chaînons, les atomes d'éléments de cycle dudit hétérocycle monocyclique ou bicyclique comprenant outre des atomes de carbone en outre 1, 2, 3 ou 4 hétéroatomes choisis parmi N, O et S en tant qu'atomes d'éléments de cycle à la condition que l'hétérocycle ne puisse pas contenir 2 atomes contigus choisis parmi O et S ; et le groupe hétéroaryle dans le groupe hétéroaryl-C₁-C₄-alkyle étant un hétérocycle aromatique à 5 ou 6 chaînons, les atomes d'éléments de cycle du cycle hétérocyclique comprenant outre des atomes de carbone 1, 2, 3 ou 4 hétéroatomes choisis parmi N, O, et S en tant qu'atomes d'éléments de cycle à la condition que l'hétérocycle ne puisse pas contenir 2 atomes contigus choisis parmi O et S ; et l'un quelconque parmi les groupes aliphatiques ou cycliques mentionnés ci-dessus étant non substitués ou substitués par 1, 2, 3, ou jusqu'au nombre maximal possible de groupes identiques ou différents R^{1a} ; ou
R¹ et R², conjointement avec l'atome d'azote auquel ils sont fixés, formant un hétérocycle à 3 à 10 chaînons monocyclique ou bicyclique saturé ou partiellement insaturé , l'hétérocycle comprenant outre un atome d'azote et un ou plusieurs atomes de carbone aucun autre hétéroatome ou 1, 2 ou 3 hétéroatomes supplémentaires indépendamment choisis parmi N, O, et S en tant qu'atomes d'éléments de cycle à la condition que l'hétérocycle ne puisse pas contenir 2 atomes contigus choisis parmi O et S ; et l'hétérocycle étant non substitué ou substitué par 1, 2, 3, 4, ou jusqu'au nombre maximal possible de groupes identiques ou différents R^{1a} ;
R^{1a} étant halogène, oxo, cyano, NO₂, OH, SH, NH₂, C₁-C₆-alkyle, C₁-C₆-halogénoalkyle, C₁-C₆-alcoxy, C₁-C₆-halogénoalcoxy, C₁-C₆-alkylthio, C₁-C₆-halogénoalkylthio, C₃-C₈-cycloalkyle, -NHSO₂-C₁-C₄-alkyle, -(C=O)-C₁-C₄-alkyle, C(=O)-C₁-C₄-alkyle, C₁-C₆-alkylsulfonyle, hydroxyC₁-C₄-alkyle, -C(=O)-NH₂, -C(=O)-NH(C₁-C₄-alkyle), C₁-C₄-alkylthio-C₁-C₄-alkyle, aminoC₁-C₄-alkyle, C₁-C₄-alkylamino-C₁-C₄-alkyle, diC₁-C₄-alkylamino-C₁-C₄-alkyle, aminocarbonyl-C₁-C₄-alkyle ou C₁-C₄-alcoxy-C₁-C₄-alkyle ;
R² étant hydrogène, C₁-C₆-alkyle, C₂-C₆-alcényle, C₂-C₆-alcynyle, C₁-C₆-alcoxy, C₃-C₁₁-cycloalkyle, -C(=O)H, - C(=O)-C₁-C₆-alkyle, -C(=O)-C₃-C₁₁-cycloalkyle ou -C(=O)-OC₁-C₆-alkyle ; et l'un quelconque des groupes aliphatiques ou cycliques dans R² étant non substitués ou substitués par 1, 2, 3 ou jusqu'au nombre maximal possible de radicaux identiques ou différents choisis dans le groupe constitué par halogène, hydroxy, oxo, cyano, C₁-C₆-alkyle, C₁-C₆-alcoxy et C₃-C₁₁-cycloalkyle ; pour obtenir un composé de formule IV

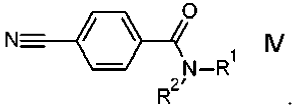

16. Procédé selon la revendication 15, comprenant en outre la mise en réaction du composé de formule IV pour obtenir un composé de formule V

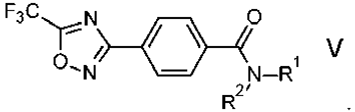

17. Procédé selon la revendication 16, comprenant en outre l'étape de mise en réaction du composé de formule V pour obtenir un composé de formule VI

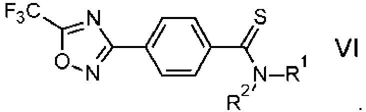

18. Procédé selon l'une quelconque des revendications 14 à 17, dans les composés de formule III, IV, V et VI
R¹ étant méthyle, 2-méthoxyiminoéthyle, bicyclo[1.1.1]pentan-1-yle, 2-fluoro-phényle, 4-fluoro-phényle ou 2,4-difluorophényle ; en particulier méthyle ou 2-fluoro-phényle ; et
R² étant hydrogène.
